Europäisches Patentamt

European Patent Office

Office européen des brevets

(11) Veröffentlichungsnummer: **0 211 178**
**B1**

(12) **EUROPÄISCHE PATENTSCHRIFT**

(45) Veröffentlichungstag der Patentschrift:
**19.04.89**

(21) Anmeldenummer: **86107677.6**

(22) Anmeldetag: **05.06.86**

(51) Int. Cl.⁴: **B01D 53/34**, C07C 45/80

(54) Verfahren zur Entfernung von n-Butyraldehyd aus Gasströmen.

(30) Priorität: **06.08.85 DE 3528124**

(43) Veröffentlichungstag der Anmeldung:
**25.02.87 Patentblatt 87/9**

(45) Bekanntmachung des Hinweises auf die Patenterteilung:
**19.04.89 Patentblatt 89/16**

(84) Benannte Vertragsstaaten:
**DE FR GB**

(56) Entgegenhaltungen:
**DE-C- 870 841**
**FR-A- 2 126 412**

(73) Patentinhaber: **HÜLS AKTIENGESELLSCHAFT,
Patentabteilung / PB 15 - Postfach 13 20,
D-4370 Marl 1 (DE)**

(72) Erfinder: **Möller, Eckhard, Dr., Guido-Heiland-Strasse 8,
D-4370 Marl (DE)**
Erfinder: **Müller, Wolfgang Hans Eduard, Dr.,
Kaspar-Grove-Strasse 32, D-4370 Marl (DE)**
Erfinder: **zur Hausen, Manfred, Dr., Hoechster Strasse 1,
D-4370 Marl (DE)**

**Beschreibung**

Die Erfindung betrifft ein Verfahren zur Entfernung von n-Butyraldehyd aus Gasströmen. Es ist gekennzeichnet durch eine Wäsche mit verdünnter, wäßriger Natronlauge und/oder Kalilauge in einer Stoffaustauschapparatur.

n-Butyraldehyd enthaltende Gasströme treten bei der Produktion von n-Butyraldehyd auf. Auch bei der Destillation und Kondensation von n-Butyraldehyd, beim Befüllen von Tanks mit n-Butyraldehyd oder bei der Anwendung von n-Butyraldehyd oder n-Butyraldehyd enthaltenden Flüssigkeiten befindet sich n-Butyraldehyd in der Abluft.

Bevor die Gasströme in die Umgebungsluft gelangen, müssen sie weitestgehend von n-Butyraldehyd gereinigt werden, um gesetzliche Bestimmungen und sonstige Auflagen zum Schutz der Beschäftigten und der Umwelt zu erfüllen. So wird n-Butyraldehyd nach der Technischen Anleitung zur Reinhaltung der Luft (TA Luft) in Klasse 2, manchmal jedoch auch wegen der leichten Oxidierbarkeit zu Buttersäure in Klasse 1 eingestuft. Nach Klasse 2 dürfen Abgabewerte von 3 kg/h und nach Klasse 1 von 0,1 kg/h nicht überschritten werden.

Nach DE-AS 1 294 950 können dampfförmige Aldehyde durch Absorption an hochsiedende Lösemittel aus Abgasen abgetrennt werden.

Nach DE-AS 2 209 098 können Aldehyde mit Hilfe einer Sulfit/Bisulfit-Lösung bei pH 6 bis 11 durch Bisulfit-Addition als salzartige Verbindungen ausgefällt werden.

Ferner ist die Aldolkondensation von n-Butyraldehyd zu 2-Ethylhexenal in der Gasphase durch Umsetzung an festen Katalysatoren nach H.E. Swift et al., Journal of Catalysis 15, 407–416 (1969) bekannt. Die Reaktion erfolgt hier bei einer Temperatur von 250°C mit Umsätzen von kaum mehr als 50% und bei schneller Inaktivierung der Kontakte.

Nach US-PS 2 684 385 kann für die Aldolkondensation von dampfförmigem n-Butyraldehyd mit flüssiger Natronlauge eine extraktive Destillation durchgeführt werden. Dazu muß n-Butyraldehyd bis zum Siedepunkt von 75°C erhitzt werden. Dieses Verfahren ist, obwohl eine Extraktion ohne Hilfsmittel nicht ausgeschlossen wird, nur mit großen Mengen an Lösungsvermittlern erfolgreich. Dabei wird für einen Umsatz von 78% eine Kolonne mit 60 Trennstufen benötigt.

Nach PL-PS 99 257 wird dampfförmiger n-Butyraldehyd bei 100°C durch eine Mischung aus 6%iger wäßriger Natronlauge und einem Lösungsvermittler, der aus alkoholreichen Kohlenwasserstoffen besteht, geleitet und dabei zu 2-Ethylhexenal kondensiert. Zur Aufarbeitung der Reaktionsprodukte wird ein Gemisch aus 2-Ethylhexenal-Wasser-Azeotrop und Alkoholen destilliert, das anschließend aufgetrennt werden muß.

Die bekannten Verfahren betreffen die Aldolkondensation von dampfförmigem n-Butyraldehyd zu 2-Ethylhexenal. Sie benötigen hohe Temperaturen und bei Verwendung wäßriger Laugen zusätzlich Lösungsvermittler, die teilweise die Isolierung von 2-Ethylhexenal als Wertprodukt erschweren. Ein Verfahren, bei dem n-Butyraldehyd in Abwesenheit von Lösungsvermittlern zu über 90% unter Bildung von 2-Ethylhexenal aus Gasströmen entfernt wird, ist nicht bekannt.

Der Erfindung liegt die Aufgabe zugrunde, n-Butyraldehyd aus Gasströmen auf eine wirtschaftlich vertretbare Art weitestgehend zu entfernen. Dabei soll n-Butyraldehyd oder ein Folgeprodukt bei geringem Aufwand als Wertprodukt gewonnen werden.

Die Aufgabe wird erfindungsgemäß dadurch gelöst, daß man die n-Butyraldehyd enthaltenden Gasströme bei einer Temperatur von 0 bis 40°C in einer einfachen Stoffaustauschapparatur mit einer verdünnten, wäßrigen Natronlauge und/oder Kalilauge in Abwesenheit von Lösungsvermittlern wäscht.

Die Gasströme, die von n-Butyraldehyd befreit werden sollen, können aus Luft, Stickstoff, Kohlenmonoxid, Wasserstoff, Methan und aus anderen, unter den gegebenen Bedingungen inerten Gasen und Gasgemischen bestehen.

Bei der Wäsche wird bevorzugt eine Temperatur von 10 bis 30°C eingestellt.

Zur Durchführung des Verfahrens können übliche Apparaturen für den flüssig/gasförmigen Stoffaustausch, wie Rührkessel, Blasensäulen oder Gegenstromapparaturen verwendet werden. Gut geeignet sind Gegenstromapparaturen wie Boden- oder Füllkörperkolonnen mit 1 bis 15 Trennstufen. Vorzugsweise werden Kolonnen mit 5 bis 10 praktischen Böden eingesetzt. Dabei bevorzugt man ganz besonders Glockenbodenkolonnen mit 5 bis 10 praktischen Böden.

Geeignet sind 0,5 bis 10%ige wäßrige Natronlaugen und/oder Kalilaugen. Vorzugsweise wird eine 2 bis 10%ige Natronlauge oder Kalilauge verwendet.

Durch das erfindungsgemäße Verfahren wird eine weitgehende Reinigung des Gasstromes von n-Butyraldehyd erreicht. n-Butyraldehyd wird zu über 90%, im allgemeinen sogar zu über 98% aus dem Gasstrom entfernt. Der apparative Aufwand ist dabei gering, da man mit einer einfachen Apparatur bei niedrigen Temperaturen mit verdünnten Laugen ohne Lösungsvermittler arbeitet.

Auf der aus der Stoffaustauschapparatur ausfließenden Lauge scheidet sich eine organische Phase ab, die leicht abgetrennt werden kann. Sie besteht zu über 90% aus 2-Ethylhexenal, das in einfacher Weise weiter gereinigt werden kann. Nach Abtrennung der organischen Phase kann die Lauge in die Apparatur zurückgeführt werden.

Das bei diesem Verfahren gewonnene 2-Ethylhexenal kann zu 2-Ethylhexanol hydriert werden, das als Weichmacher von Interesse ist.

Beispiele

Allgemeine Versuchsbedingungen: Durch eine gläserne Glockenbodenkolonne mit einem Durchmesser von 80 mm, die unter Normaldruck steht, wird ein mit n-Butyraldehyd beladener Stickstoffstrom, der die gleiche Temperatur wie die Kolonne hat, im Gegenstrom zu einer verdünnten, wäßrigen Natronlauge geführt. Die unten aus der Kolonne ausfließende Natronlauge wird, nachdem die sich bildende organische Phase abgetrennt ist, im Kreise zurückgeführt.

Die Versuchsbedingungen und Meßwerte sind in Tabelle 1 zusammengefaßt. Dabei sind erfindungsgemäße Beispiele mit Zahlen und Vergleichsbeispiele mit Buchstaben gekennzeichnet.

Tabelle 1

| Beispiel | Anzahl der praktichen Böden | Temperatur [°C] | Konzentration der Natronlauge [Gew.%] | Gasdurchsatz [Nm$^3$N$_2$/h] | Laugedurchsatz [l/h] | Anfangskonz. Gas [g n-BA/Nm$^3$] | Endkonz. Gas [g n-BA/Nm$^3$] | Abscheidung n-BA [%] |
|---|---|---|---|---|---|---|---|---|
| 1 | 1 | 23 | 10 | 203,2 | 15 | 384,2 | 23,3 | 94,62 |
| 2 | 1 | 23 | 10 | 203,2 | 15 | 809,4 | 46,0 | 95,68 |
| 3 | 1 | 24 | 10 | 201,2 | 15 | 1751,1 | 120,8 | 96,73 |
| 4 | 5 | 23 | 10 | 255,8 | 15 | 447,7 | 8,49 | 98,36 |
| 5 | 5 | 23 | 10 | 216,6 | 15 | 912,8 | 6,03 | 99,53 |
| 6 | 5 | 26 | 10 | 205,4 | 15 | 1634,0 | 21,57 | 99,35 |
| 7 | 10 | 24 | 10 | 199,6 | 15 | 465,9 | 2,0 | 99,63 |
| 8 | 10 | 24 | 10 | 206,7 | 15 | 907,9 | 1,3 | 99,90 |
| 9 | 10 | 26 | 10 | 203,2 | 15 | 1733,7 | 1,5 | 99,96 |
| A | 1 | 50 | 10 | 200,8 | 15 | 435,4 | 156,3 | 67,37 |
| 10 | 5 | 34 | 10 | 323,8 | 15 | 1819,0 | 64,82 | 98,42 |
| B | 10 | 45 | 10 | 140,3 | 15 | 482,4 | 82,6 | 85,06 |
| 11 | 5 | 22 | 2 | 214,0 | 15 | 445,3 | 5,88 | 98,86 |
| 12 | 5 | 27 | 2 | 202,8 | 15 | 908,0 | 11,53 | 99,09 |
| 13 | 5 | 28 | 2 | 193,6 | 15 | 1625,0 | 19,81 | 99,39 |

n-BA = n-Butyraldehyd

## Patentansprüche

1. Verfahren zur Entfernung von n-Butyraldehyd aus Gasströmen, dadurch gekennzeichnet, daß die Gasströme bei einer Temperatur von 0 bis 40°C mit einer 0,5 bis 10%igen wäßrigen Natronlauge und/oder Kalilauge in Abwesenheit von Lösungsvermittlern gewaschen werden.

2. Verfahren nach Anspruch 1, dadurch gekennzeichnet, daß bei einer Temperatur von 10 bis 30°C gewaschen wird.

3. Verfahren nach den Ansprüchen 1 und 2, dadurch gekennzeichnet, daß eine 2 bis 10%ige wäßrige Natronlauge und/oder Kalilauge verwendet wird.

4. Verfahren nach den Ansprüchen 1 bis 3, dadurch gekennzeichnet, daß die Lauge im Kreise geführt wird.

5. Verfahren nach den Ansprüchen 1 bis 4, dadurch gekennzeichnet, daß man im Gegenstrom wäscht.

6. Verfahren nach Anspruch 5, dadurch gekennzeichnet, daß man die Gasströme in einer Kolonne mit 5 bis 10 praktischen Böden wäscht.

7. Verfahren nach Anspruch 6, dadurch gekennzeichnet, daß man die Gasströme in einer Glockenbodenkolonne wäscht.

## Claims

1. A process for removing n-butyraldehyde from a gas stream, characterized in that the gas stream is washed at a temperature of from 0 to 40°C with 0.5 to 10% strength aqueous sodium hydroxide solution and/or potassium hydroxide solution in the absence of solubilizers.

2. A process according to claim 1, characterized in that the washing is carried out at a temperature of from 10 to 30°C.

3. A process according to either of claims 1 and 2, characterized in that 2 to 10% strength aqueous sodium hydroxide solution and/or potassium hydroxide solution is used.

4. A process according to any of claims 1 to 3, characterized in that the hydroxide solution is circulated.

5. A process according to any of claims 1 to 4, characterized in that the washing is carried out in countercurrent.

6. A process according to claim 5, characterized in that the gas stream is washed in a column having 5 to 10 real plates.

7. A process according to claim 6, characterized in that the gas stream is washed in a bubble-cap plate column.

## Revendications

1. Procédé pour éliminer le n-butyraldéhyde de courants gazeux, caractérisé par le fait qu'en l'absence de solubilisants on lave les courants gazeux, à une température de zéro à 40°C, avec une solution aqueuse d'hydroxyde de sodium et/ou de potassium d'une teneur de 0,5 à 10%.

2. Procédé selon la revendication 1, caractérisé par le fait que l'on effectue le lavage à une température de 10 à 30°C.

3. Procédé selon les revendications 1 et 2, caractérisé par le fait que l'on utilise une solution aqueuse d'hydroxyde de sodium et/ou de potassium d'une teneur de 2 à 10%.

4. Procédé selon les revendications 1 à 3, caractérisé par le fait que l'on fait circuler la solution d'hydroxyde.

5. Procédé selon les revendications 1 à 4, caractérisé par le fait que l'on effectue le lavage à contre-courant.

6. Procédé selon la revendication 5, caractérisé par le fait que l'on lave les courants gazeux dans une colonne comportant de 5 à 10 plateaux pratiques.

7. Procédé selon la revendication 6, caractérisé par le fait que l'on lave les courants gazeux dans une colonne à plateaux en cloche.